# EUROPEAN PATENT APPLICATION

(11) **EP 4 230 035 A1**
(43) Date of publication of application: **23.08.2023**
(21) Application number: 22157390.0
(22) Date of filing: 18.02.2022
(51) Int. Cl.: A01M 1/00, A01K 67/00

(54) **NON-RADIATIVE AND NON-GM STERILE INSECT TECHNOLOGY (SIT) FOR THE CONTROL OF HARMFUL INSECTS**

(71) Applicant: Fraunhofer-Gesellschaft zur Förderung der angewandten Forschung e.V., 80686 München (DE)
(72) Inventor: Lee, Kwang-Zin, 35392 Giessen (DE); Vilcinskas, Andreas, 35392 Giessen (DE)
(74) Representative: Roth, Andy Stefan

(57) **Abstract**

The present technology pertains to a fundamentally new idea to sterilize male insect pests by means of a targeted heat treatment and use this as a sterile insect technique (SIT) for a species-specific biological control method that can reduce or eliminate populations of pest insects without the need of chemical pesticides or genetically-modified insects. In particular, the present technology pertains to methods for the suppression of a target pest population comprising introducing a plurality of heat-treated sterile males of the target pestto the target pest population, wherein the males are heat-treated for a period of time.

## Description

### BACKGROUND

Insects, fungi, nematodes, protozoa, bacteria and viruses are responsible for widespread damage to crops and animals worldwide with enormous concomitant economic consequences. In addition, many human and veterinary health issues are associated with the spread of disease by insects and other pests.

For example spotted wing Drosophila, *D. suzukii,* is a pest of many small and soft fruit, including cherries, raspberries, blackberries, blueberries, strawberries, peaches, grapes, and others. It damages these fruit by using its heavily sclerotized and serrated ovipositor to pierce fruit and lay eggs inside them. Most of the damage caused by *D. suzukii* is a result of larvae feeding on fruit flesh. However, the insertion of the prominent ovipositor into the skin of the fruit can also cause physical damage to the fruit, as it provides access to secondary infections of pathogens - such as fungi, yeasts and bacteria - that may cause faster fruit deterioration and further losses. These damages can result in severe crop losses, and the implications for exporting producers may also be severe, depending on quarantine regulations.

A native of eastern and southeastern Asia, *D. suzukii* has invasively spread in the last several decades, and has been recorded in China, India, Italy, Spain, Russia and a number of other countries. *D. suzukii* has also rapidly invaded the U.S. - initially found in California in 2008, it has spread to much of the Pacific Coast, to the East Coast, as well as to north central and interior U.S. - and poses a significant threat to fruit industries there.

In order to reduce pest-inflicted damage, resources have been devoted to the development and deployment of pesticides, which control pest populations by killing target pests. Although pesticides are in many cases effective, they are known to be also toxic to life forms other than the target organism, which has important environmental consequences. Accordingly, there has been a focus on development of alternative, more biological, pest control methods that do not involve pesticides.

The sterile insect technique (SIT) is a species-specific biological control method that can reduce or eliminate populations of pest insects without the need of chemical pesticides. The technique requires the release of large numbers of sterilized males, which then compete with wild males for female mates to reduce the population of the pest insect. Typically used to sterilize males for SIT are radiation or chemosterilants, but these treatments can decrease their ability to compete for mates, which often necessitates the production and release of many more sterile males to increase efficacy. SIT is considered more effective if females are not released, as sterile female insects can still damage crops or transmit disease. Genetic sexing techniques have been developed for some insects, such as the medfly, to preferentially eliminate females before they mature. These typically involve chromosomal translocations that link the male-determining chromosome to a dominant selectable marker, while the females are homozygous for a recessive deleterious gene. Unfortunately, these translocations tend to break down when insects are mass-reared due to male chromosomal recombination..

Conventional SIT has been previously used to control mosquitoes, but not in an extensive way due to the limited competitiveness of the sterile males. New transgenic methods, using genetically altered insects that carry and spread deleterious genes are being developed, but the release of genetically-modified insects may be prohibited or delayed in many communities/countries until public opinion and regulatory issues are fully considered.

### SUMMARY OF THE DISCLORE

A fundamentally new idea is to sterilize male insect pests by means of a targeted heat treatment and use this as a sterile insect technique (SIT) for a species-specific biological control method that can reduce or eliminate populations of pest insects without the need of chemical pesticides or genetically-modified insects.

Therefore, the present disclosure shows that insect pests are sterilized by heat treatment (e.g. between 28 °C and 31 °C) and that this sterile insect technique (SIT) is applicable against several insect species, in particular against the cherry vinegar fly *D. suzukii* and other insect pests (e.g. mosquitoes).

In particular, the present disclosure provides methods for biological control of an insect, in particular an insect population. In one embodiment, the method includes releasing a population of modified (heat-treated) pests like insects into an environment, wherein the number of progenies in the next generation is reduced.

In an aspect of the present disclosure, the present invention relates to a method for controlling a pest population in a target region comprising release of a plurality of heat-sterilized males to the target region.

Further aspects of the present disclosure pertains to mass rearing of heat-sterilized *D. suzukii* males in an appropriate incubator with constant 30 °C heating. The heat-sterilized *D. suzukii* males can be separated from the female flies manually by visual inspection under the stereomicroscope.

### Brief Description of the Figures

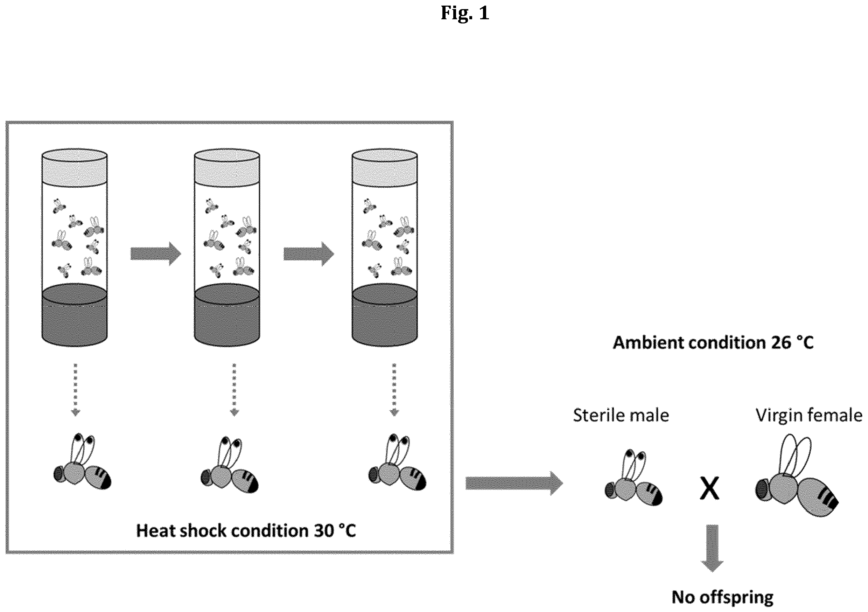

Figure 1 is a scheme of an experimental setup for producing heat-sterilized *D. suzukii* males and subsequent single pair mating.

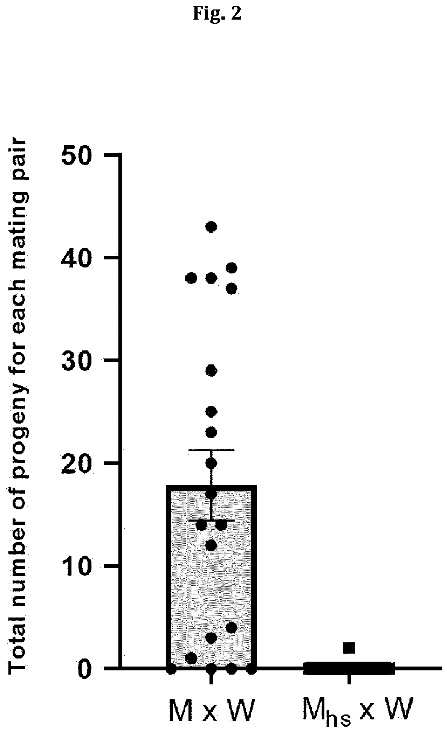

Figure 2 is a graph showing the number of offspring from single matings of conventional males vs. males incubated under heat stress.

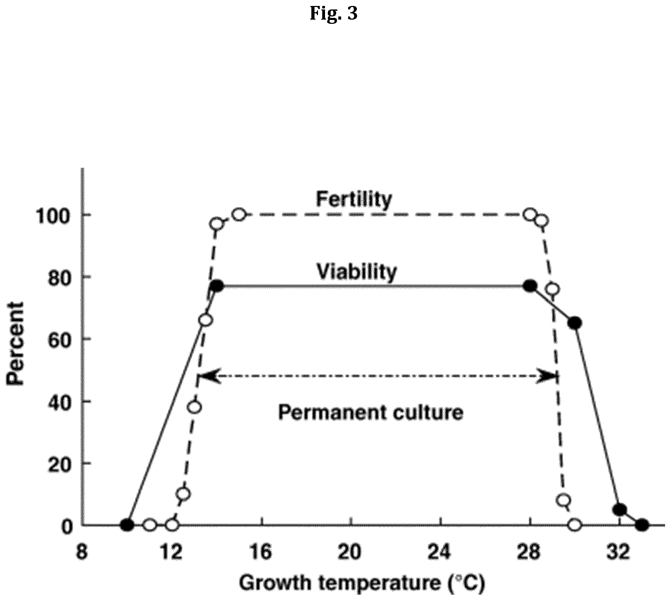

Figure 3 is a graph showing a comparison of reaction norms for viability and male fertility according to growth temperature in a temperate strain of *D. melanogaster (*J of Evolutionary Biology, Volume: 18, Issue: 4, Pages: 838-846, First published: 11 May 2005, DOI: (10.1111/j.1420-9101.2005.00914.x)
Figure 4 is a graph showing the significant reduction (p=0.0312, unpaired t-test) of progeny (control=750 hatched adults vs heat-treated=477 hatched adults) after introducing a plurality of heat-treated sterile *D. suzukii* males of the target pest to a *D*. *suzukii* target pest population.

### DETAILED DESCRIPTION OF ILLUSTRITIVE EMBODIMENTS

The present invention relates to methods of controlling pest populations through the sterilization of male insect pests by means of a targeted heat treatment.

Provided herein are further methods of producing insects that are sterile and/or have reduced fecundity, such as mosquitoes or *Drosophila* flies like *D. suzukii,.* Such insects may be used in sterile insect technique (SIT) programs to eradicate pests and thereby reduce disease transmission and/or economic losses caused by pests.

As mentioned above, the present disclosure pertains to methods to control insect populations by SIT and this has been seen as an effective, species-specific and environmentally friendly method for controlling pest populations. The SIT method according to the present disclosure creates sterile male insects through the use of heat treatment and releases them into natural habitats where the males would instinctively look for natural females to mate with. When a huge number of sterile males are released over an extended period of time, it can cause the natural insect population to collapse, or even become extinct.

As mentioned above, SIT is a biological method of controlling pest insects by releasing a large number of sterile males into a locality, where they compete with wild males for females and so can effectively reduce or eliminate the pest population. Existing methods of producing sterile males for pest insects have traditionally relied upon the use of radiation or broad-spectrum chemosterilants to sterilize males, but these methods typically render the males weak and not highly competitive when they are released. Some new methods that rely on producing genetically-modified mosquitoes have been developed, but these approaches require the release of such insects into the environment that can spread the gene into the population, which may have unforeseen impacts on the population beyond the region of concern.

The present disclosure pertains to methods for the suppression/control of a target pest population comprising introducing a plurality of heat-treated sterile males of the target pest to the target pest population, wherein the males are heat-treated for a period of time.

In particular, the sterile males of the target pest are heat-treated with a temperature between 25 °C and 50 °C, in particular between 25 °C and 40 °C, in particular between 28°C and 32°C, in particular between 29°C and 31°C in particular with 30°C.

The present disclosure pertains in particular to a method for the suppression/control of a target pest population, in particular of *D. suzukii,* comprising the introduction of a plurality of heat-treated sterile males of the target pest to the target pest population, wherein the males are heat-treated for a period of time and with an temperature between 28°C and 31°C, in particular with 30°C.

I view of *D. suzukii* , increasing the incubation temperature to between 28 °C and 31°C, in particular to 30 °C leads to male sterility in the insects due to disruption of spermatogenesis, such as higher spermatid mortality rate, cytological abnormalities in the sperm head and inactivation of cyst elongation.

In some advantageous embodiments, the period of time is between 30 and 100 h, in particular between 50 and 80 h, in particular about 72 h. In further embodiments, the plurality of heat-treated sterile males of the target pest comprises at least 50 individual males.

In one advantageous embodiment, the plurality of heat-treated sterile males are provided in a first population of the target pest, wherein the first target pest population is heat-treated for a period of time and with an temperature between 28°C and 31°C, in particular with 30°C; and applying the first target pest population to a desired area, wherein the desired area contains a second target pest population, thereby controlling, suppressing or eliminating the second target pest population in the desired area. In particular, the first insect population comprises males and females of the target pest. In some advantageous embodiments, the first population of the target pest comprises only males.

In some advantageous embodiments of the present disclosure, the target pests are insects that may be modified using the methods described herein include, but are not limited to pests that act as vectors for viral, bacterial, and/or parasite-based diseases; and/or inflict damage on crops, fruit, vegetables, animals, and/or humans. In one embodiment, an insect is a member of the family *Culicidae.* Examples of such insects include mosquitoes, such as *Aedes spp.* (e.g., *A. aegypti, A. albopictus,* and *A. vexans), Anopheles spp.* (e.g., *A. gambiae, A. farauti, A. quadrimaculatus, A. stephensi, A. arabiensis*), and *Culex spp.* (e.g., *C*. *pipiens, C*. *quinquefasciatus,* and *C*. *tarsalis*). In one embodiment, an insect is a member of the family *Tephritidae.* Examples of such insects include fruit flies such as *Ceratitis capitata* (commonly referred to as the Medfly), *Anastrepha spp.* (e.g. *A. ludens, A. suspense, A. oblique*) and *Bactrocera spp.* (e.g., *B. tryoni, B. dorsalis,* and *B. oleae, B. cucurbitae*). In another embodiment, an insect is a medical or animal pest that is a member of the Order Diptera, such as a screw worm fly (*Cochliomyia hominivorax*), or tsetse fly (*Glossina spp, such as G. palpalis, G.fuscipes, G. morsitans, G. tachinoides, G. longipalpis, G. fusca, G. tabaniformis, G. brevipalpis, G. vanhoofi, G. austeni*). In one embodiment, an insect is a member of the Order Lepidoptera (e.g. member of the Lymantriidae family, e.g. *Orgyia anartoides*) or of the *family Tortricidae,* (e.g. *Cydia pomonella*).

However, suitably the pest species is an insect of economic and medical importance. In particular, the controlled insects are flies, in particular belonging to the family *Drosophilidae,* in particular belonging to the genus Drosophila, in particular *D. suzukii.*

Furthermore, the pest species is selected from Medfly (*Ceratitis capitata*), *Aedes aegypti,* Anopheline mosquitoes such as *Anopheles gambiae, Bactrocera oleae* (olive fly), other tephritids, whiteflies and aphids, for example.

As used herein, a "population of target pests" or a "pest population" may refer to a group of given species of a pest which infests a particular crop or vertebrate in a given geographical area. Alternatively, it may refer to all pests infesting any crop or vertebrate in a geographical area, or a given species without reference to any geographical limitation, or a population of pests which is responsible for a human or veterinary health problem, such as the spread of malaria. Target pests are the individual members of the population of pests.

With the methods of the present disclosure a target pest population may be controlled, in particular in nature.

"Control" as used herein refers to the limitation, prevention or reduction of population growth, i.e., by at least about 10% per generation, preferably at least about 50%, 80%, or even up to and including 100% of the pest population. Preferably, this is achieved by reducing the number of viable young individuals for example by reducing the number of eggs hatching. Advantageously, the population of pests is eliminated.

"Population suppression" or "suppression of a target pest population" is used to describe the reduction of numbers in a population. In particular, population suppression is measured by a reduction in the number of hatched eggs or living insects.

In some advantageous embodiments of the present disclosure, the males of the target pest in the P0 and/or in the F1 generation (larva, pupa and/or adult) are heat-treated at a humidity of about 65 to 80%, in particular at 70 %.

In some advantageous embodiments of the present disclosure, the plurality of heat-treated sterile males are heat-treated in a container, in particular in an incubator.

The term "and/or" means one or all of the listed elements or a combination of any two or more of the listed elements.

The words "preferred" and "preferably" refer to embodiments of the invention that may afford certain benefits under certain circumstances. However, other embodiments may also be preferred under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

Unless otherwise specified, "a", "an", "the" and "at least one" are used interchangeably and mean one or more than one.

Conditions that are "suitable" for an event to occur, or "suitable conditions" are conditions that do not prevent such events from occurring. Thus, these conditions permit, enhance, facilitate, and/or are conducive to the event

As described in David et al., 2005 (J of Evolutionary Biology, Volume: 18, Issue: 4, Pages: 838-846, First published: 11 May 2005, DOI: (10.1111/j.1420-9101.2005.00914.x), after development at 12 °C or 30 °C, a good viability is observed in *D. melanogaster,* but all males are sterile. Such an absolute thermal threshold is easily defined by keeping a large population at its developmental temperature for several weeks: no progeny will be observed. In an outbred strain, almost all males are fertile between 14 °C and 28 °C. Between 28° and 30 °C, only a part of them will be fertile. There are two general ways to estimate the proportion of fertile males. Each male may be mated with a normal female and then cross examined for progeny production. This method is time and culture-vials consuming, consequently, observation of motile sperm in the seminal vesicles after dissection is more favorable. This method has often been used in interspecific hybrid studies to monitor the restoration of male fertility when F1 fertile females are backcrossed to parental species (David et al., 1976). It is however known that, in such cases, not all the males classified as fertile are able to produce progeny.

In the experiments concerning heat-induced sterility, a strong though not absolute correlation between progeny production and presence of motile sperm was found. In other words, male dissection data are a convenient predictor of male fertility, and a sample of 50 males is generally sufficient for estimating a percentage.

For any method disclosed herein that includes discrete steps, the steps may be conducted in any feasible order. And, as appropriate, any combination of two or more steps may be conducted simultaneously.

In some advantageous embodiments of the present disclosure, the methods according to the present disclosure comprise that
a) the parental generation P0 of the heat-treated sterile males of the target pest is heat-treated for a period of time;
b) the eggs, the larval stages, the pupae and the hatched adults from the parental generation P0 (the filial generation F1) are further heat-treated for a period of time;
c) a population of adult heat-treated sterile males were selected from the heat-treated filial generation F1 and introduced to the target pest population.

In some advantageous embodiments of the present disclosure, the methods according to the present disclosure comprise that
d) the parental generation P0 of the heat-treated sterile males of the target pest, in particular wherein the target pest is *D. suzukii,* is heat-treated for a period of time and with an temperature between 28°C and 31°C, in particular with 30°C;
e) from the parental generation P0, the eggs, the larval stages and the pupae of the hatched filial generation F1 of the parental generation P0 were maintained at a temperature between 28°C and 31°C, in particular at 30°C;
f) a population of heat-treated sterile males were selected from the heat-treated filial generation F1 and introduced to the target pest population.

For the two above mentioned embodiments, the period of time of the heat-treatment of steps a) and b) may be between 10 to 18 days in total.

Accordingly, provided herein are methods for producing modified insects. As used herein, the term "modified" refers to an insect that has been heat-treated. A modified insect may be at any developmental stage, such as larva, pupa or adult. In one embodiment, a modified insect is a larva or pupa that will develop into an adult that has reduced fertility, reduced fecundity, or a combination thereof. In one embodiment, a modified insect is an adult and has reduced fertility, reduced fecundity, or a combination thereof.

The above summary of the present invention is not intended to describe each disclosed embodiment or every implementation of the present invention. The description that follows more particularly exemplifies illustrative embodiments. In several places throughout the application, guidance is provided through lists stating which examples can be used in various combinations.

### Examples

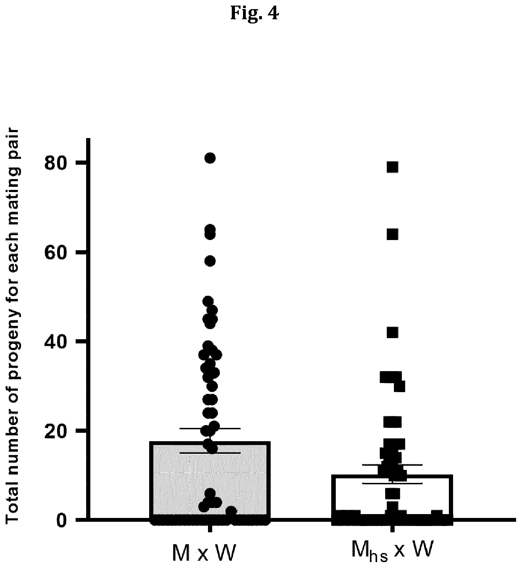

1)
   In a first example of the methods according to the present disclosure, a population of *Drosophila suzukii* comprising of at least 50 males was heat-treated for 72 hours at 30°C.
   Materials and methods:
      - Start of heat induction of adult flies (3 days post eclosure)
      - Sterilization procedure 30°C, 70% humidity for 72 h
      - control condition: 26°C, 65% humidity for 72 h
      - First pairing of single mating at 26°C, 65% humidity
      - Two transfers of the single mating pairs
   As shown in Fig. 4, the method according to the present disclosure leads to a significant reduction (p=0.0312, unpaired t-test) of progeny in such single mating experiments (control=750 hatched adults vs heat-treated=477 hatched adults).
*2)*
   *Drosophila suzukii* flies comprising of males and females were maintained in 2.5 cm diameter vials in an incubator at 30 °C and 70% humidity for 24 hours. The vials contained feeding media consisting of 10.8% (w/v) soybean and cornmeal mix, 0.8% (w/v) agar, 8% (w/v) malt, 2.2% (w/v) molasses, 1.5% (w/v) nipagin, and 0.625% propionic acid. The parental flies are transferred daily to fresh vials. Male progeny from the parental generation which has completed their life cycle at 30 °C were collected by visual inspection under the stereomicroscope from the vials.

The sterility was assessed by setting up single mating of heat-sterilized males with normal (26 °C at 65% humidity) raised virgin females at ambient conditions (26 °C, 65% humidity). As control, single mating of normal raised males with normal raised virgin females were set up at ambient conditions (26 °C, 65% humidity). The single mating pairs were transferred to fresh vials every six days. Twenty single mating pairs were observed per condition. It is very important to keep the heat shock temperature at 30 °C. When experiments were conducted at 32 °C, all flies died.

As a result, from 20 single mating experiments with heat-sterilized males, just two vials had low number of progeny (n=6), whereas the control mating experiments, most of the vials produced progeny (16 out of 20 vials, totaling in n=527 progeny).

## Claims

1. A method for the suppression of a target pest population comprising introducing a plurality of heat-treated sterile males of the target pest to the target pest population, wherein the males are heat-treated for a period of time.

2. The method according to claim 1, wherein the period of time is between 30 and 100 h, in particular between 50 and 80 h, in particular about 72 h.

3. The method according to any one of claims 1 to 2, wherein the sterile males of the target pest are heat-treated with a temperature between 25 °C and 50 °C, in particular between 25 °C and 40 °C, in particular between 28°C and 32°C, in particular between 29°C and 31°C in particular with 30°C.

4. The method according to any one of claims 1 to 3, wherein the plurality of heat-treated sterile males of the target pest comprises at least 50 individual males.

5. The method according to any one of claims 1 to 4, wherein , the plurality of heat-treated sterile males are provided in a first population of the target pest, wherein the first target pest population is heat-treated for a period of time and with an temperature between 28°C and 31°C, in particular with 30°C; and applying the first target pest population to a desired area, wherein the desired area contains a second target pest population, thereby controlling, suppressing or eliminating the second target pest population in the desired area.

6. The method according to claim 5, wherein the first target pest population comprises males and females of the target pest, in particular the first target pest population comprises only males.

7. The method according to any one of claims 1 to 6, wherein the plurality of heat-treated sterile males are heat-treated in a container, in particular in an incubator.

8. The method according to any one of claims 1 to 7, wherein
a) the parental generation P0 of the heat-treated sterile males of the target pest is heat-treated for a period of time;
b) the eggs, the larval stages, the pupae and the hatched adults from the parental generation P0 (the filial generation F1) are further heat-treated for a period of time;
c) a population of adult heat-treated sterile males were selected from the heat-treated filial generation F1 and introduced to the target pest population.

9. The method according to any one of claims 1 to 8, wherein the target pest is an insect, in particular flies, in particular flies belonging to the family *Drosophilidae,* in particular belonging to the genus Drosophila, in particular *D. suzukii.*

10. The method according to claim 9, wherein the sterile males of the target pest are heat-treated with a temperature between 28°C and 32°C, in particular between 29°C and 31°C in particular with 30°C.

11. The method according to any one of claims 8 to 10, wherein the period of time of the heat-treatment of steps a) and b) is between 10 to 18 days in total.

12. The method according to any one of claims 1 to 11, wherein the males of the target pest are heat-treated at a humidity of about 65 to 80%, in particular at 70 %.
